# EUROPEAN PATENT SPECIFICATION

(11) **EP 0 533 089 B1**
(45) Date of publication and mention of the grant of the patent: **29.11.1995**
(21) Application number: 92115686.5
(22) Date of filing: 14.09.1992
(51) Int. Cl.: C07C 409/16, C08L 23/02, C08K 5/14

(54) **Liquid peroxidic compositions**
Flüssige Peroxidzusammensetzungen
Compositions peroxydiques liquides

(30) Priority: 19.09.1991 IT MI912492
(43) Date of publication of application: 24.03.1993
(73) Proprietor: ELF ATOCHEM ITALIA S.r.l., 20159 Milano (IT)
(72) Inventor: Merenda, Michele, I-15047 Alessandria (IT); De Franco, Rita, I-27058 Voghera (PV) (IT); Scotti, Carlo, I-27058 Voghera (PV) (IT)
(74) Representative: Simeoni, Lucio, Dr.

(56) References cited:
- DE-A- 2 942 362
- GB-A- 2 017 110

## Description

The present invention refers to liquid peroxidic compositions of bis(alpha-t-butyl-peroxy-isopropyl)-benzene.

It is well known that the above compound, known as PEROXIMON F, is used in the vulcanization of polymers, e.g. polyethylene, elastomers, e.g. as ethylene-propylene (EP) or containing also units deriving from a dienic monomer (EPDM) etc. See, for example, USP 3,764,628 and Italian Patent No. 651,288. But its use in the vulcanization of polymers is limited, being a compound solid at room temperature. In order to obtain a homogenous vulcanized compound it is therefore necessary to maintain PEROXIMON F in the molten state to have a uniform dosage.
This operation has the disadvantage, in particular, to impart losses in peroxidic content and the introduction of impurities.

It is known in the art to overcome these drawbacks by introducing additives having the function to lower the melting point under the room temperature.

However, this solution presents the drawback to reduce the efficiency of the vulcanization since the additives are inactive substances.

It is known from Italian Patent No. 1,209.130 to prepare liquid composition of PEROXIMON F by solubilizing said bisperoxide with isopropylcumyl-t-butylperoxide. However the amount of cumylperoxide is very high: 75-90% by weight. Furthermore high quantities of cumylperoxide are required in order to obtain, high MH values (see below) as requested in the vulcanization of the above-cited polymers, in particular polyethylene. This represents a drawback from the industrial viewpoint.

Further the composition is liquid at room temperature of 23°C. This is a drawback since it is desirable to have compositions which are liquid at room temperature up to 15°C so permitting a higher use range.

In this way it is avoided to melt the compound at a temperature lower than 23°C since this gives rise to the abovementioned drawbacks as, for example, the introduction of impurities in the system which brings to a poor vulcanization in the finished article.

Other liquid peroxidic compositions are known from USP 4,202,790 and 4,239,644 where PEROXIMON F is substituted with DCP and is solubilized with cumylisopropyl-cumylperoxide or peroxides of similar formula. But also these peroxidic compositions present the drawback to be liquid only at 23°C, giving the same disadvantages said above.

From USP 4,450,302 liquid peroxidic compositions are also known, obtained from PEROXIMON F and DCP (dicumylperoxide) and t.butyl-cumyl-peroxide (PEROXIMON 166) which are liquid at very low temperatures, also lower than 5°C. Nevertheless these compositions present the drawback to be too volatile at a temperature of 15°C, due to the use of PEROXIMON 166.

Therefore it was desirable to have available liquid compositions of PEROXIMON F having the optimum balance of the following properties:
- to be liquid at room temperature, also at 15°C or lower;
- low volatility, so to permit that the formulates with the inert materials necessary for the vulcanization, as kaolin, calcium carbonate or master in elastomers or polymers in general, can be stocked for long periods without losses in peroxidic content;
- to show an optimum MH value (maximum value of the torque couple) combined with the optimum scorching time (see the definitions below) such to obtain a high vulcanization and a uniform cross-linking:
The Applicant has surprisingly found that it is possible to obtain the combination of the above properties by the following composition consisting essentially of:
- from 65 to 95 parts by weight of bis(alpha-t-amyl-peroxy-isopropyl)-benzene (PEROXIMON 180) having the formula: 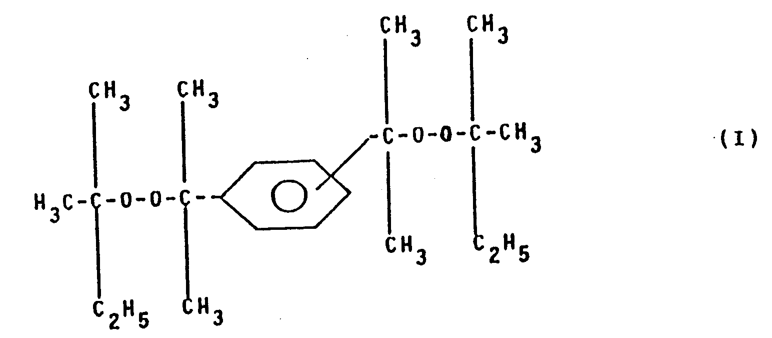
- from 5 to 35 parts by weight of bis(alpha-t-butyl-peroxy-isopropyl)-benzene (PEROXIMON F) of formula: 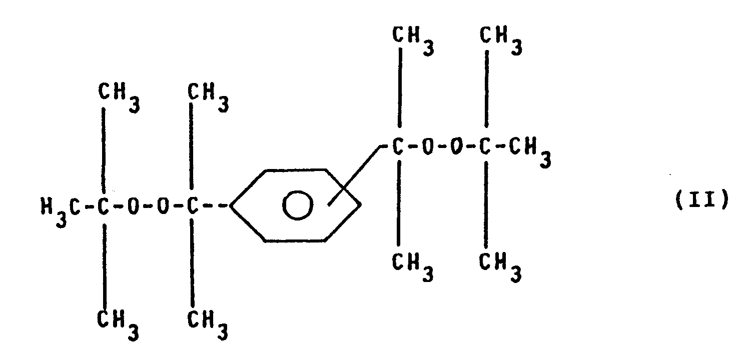 the substituting groups in (I) and (II) being in meta and/or para position with respect to the benzen ring and excluding the combinations para PEROXIMON 180/para PEROXIMON F and meta PEROXIMON 180/para PEROXIMON F; preferably the meta/para ratios range between 1 and 2.0 in (I) and between 1.5 and 2.0 in (II) when mixtures of meta/para of (I) and (II) are used.

Liquid peroxidic compositions of (I) and (II) can be prepared by mixing (I) and (II) in the indicated ratios.

The Applicant has found that the crosslinking power of the above liquid compositions, at constant peroxidic content, increases proportionally with the increase of the ratio by moles terbutyl groups/(tert.butyl groups + tert.amyl groups).

This is shown when considering the molar ratios tert.butyl groups/(tert.butyl + tert.amyl groups) with respect of the maximum value of the torque couple MH (which meaning is shown in example 1/d) obtained in the cross-linking tests of examples 1/d, 2/a where peroxidic mixtures having the same active oxygen content are obtained.

Up to a ratio of 0.27, the compositions are liquid at temperatures of 15°C and stable when stocked for a very long period.

The liquid compositions of the present invention show therefore a cross-linking power higher than PEROXIMON 180, which is liquid at room temperature but with a very low cross-linking power. Furthermore it is more advantageous from the economics to use the liquid compositions of the present invention since PEROXIMON F is obtained by cheaper reagents: t-butyl hydroperoxide instead of t-amyl hydroperoxide.
Preparation processes of (I) and (II) are well known in the art, see, for example, USP 3,658,914 and Italian Patents No. 707,597, No. 651,288 and No. 805,543 in addition to ones indicated above.

A preferred process consists in the alkylation of benzene with an excess in propylene, the preferred ratio being 1:1.5 molar, in the presence of AlCl₃ according to Friedel-Kraft reaction; if desired, the mixture containing diisopropylbenzene, prevailingly meta and para, can be rectified to obtain the two isomers separated.

Then an oxidation is carried out by known methods, for example air, oxygen etc., in order to obtain the dihydroperoxide of the diisopropylbenzene.

The so prepared dihydroperoxide is extracted by adding an aqueous solution of caustic soda and a subsequent reduction according to known techniques, for examples by sulphides, sulfites, sulphydrates to obtain the dialcohol of formula: 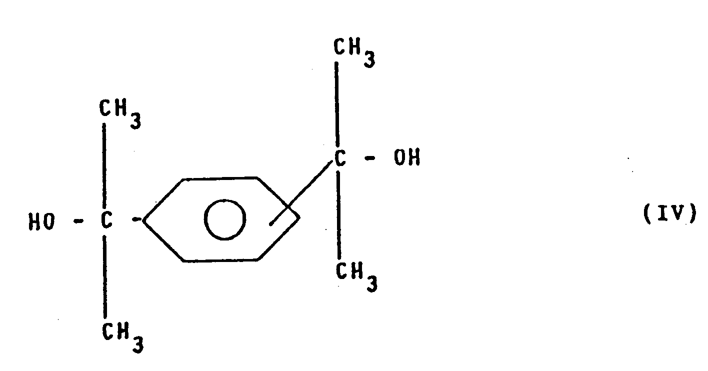
the two substituent groups of the benzen ring being in meta and/or para position.

The alcohol is separated by centrifugation or filtration of the aqueous phase.

PEROXIMON F is then obtained by reacting the dialcohols (IV) with tert.butylhydroperoxide, in hydrocarbons solution and/or ditert.butylperoxide, or in aqueous solution at 70% by weight, in the presence of one of the known acid catalysts, among then, e.g., the sulphuric, the metansulphonic, the paratoluensulphonic. The PEROXIMON F, product solid at room temperature, is obtained in meta and/or para form, depending on the starting alcohols.

The so obtained peroxidic mixture is washed, for example, by an alcaline solution; then it is concentrated to remove the low-boiling compounds, essentially hydrocarbon solvents and peroxidic by-products, such as ditert.butylperoxide; preferably by distillation under steam current or by evaporation of the solvent.

In a similar way the dialcohol (IV) can be reacted with tert.amylhydroperoxide, in hydrocarbons solution and/or in ditert.amylperoxide, so obtaining the PEROXIMON 180 (I).

It has found that binary mixtures PEROXIMON F/PEROXIMON 180 obtained by simple mixing of the two products, remain liquid at room temperature (15°C) up to a molar ratio terbutyl groups/sum of terbutyl and teramyl groups of 0.27.

Therefore the use of such mixtures is more convenient than the use of PEROXIMON 180 alone, liquid too, both for the improved cross-linking power and for the production cost: the raw material of PEROXIMON F, i.e. tert.butylhydroperoxide, being cheaper than the raw material of PEROXIMON 180, i.e. tert.amylhydroperoxide.

Polymers which can be cross-linked by the compositions of the present invention are generally elastomeric polymers.

More particularly, medium-, low-, high-density polyethylene, polybutene-1, ethylene/vinylacetate copolymers, acrylic ester/ethylene copolymers, ethylene/propylene copolymers, ethylene/butene-1 copolymers, ethylene/4-methylpentene-1 copolymers and propylene/butene-1 copolymers can be cited; and furthermore ethylene/propylene elastomeric polymers, EP or EPDM type, butylic rubber, chlorinated polyethylene and propylene/butene-1 copolymer.

It can be successfully cross-linked also mixture of two or more thermoplastic olefinic polymers or two polymers of elastomeric type, or mixture of at least an elastomeric with a thermoplastic polymer. The new compositions can be used not only for the vulcanization of compact articles obtained by extrusion, but also for the production of expanded articles (cross-linked) derived from the same materials, in particular from polystyrene containing auto-extinguishing agents (flame-extinguishing agents), furthermore the same compositions can be used as coadjuvants of polymers degradable by peroxides, for example polypropylene or poly-4-methyl-pentene-1 and as radicalic polymerization initiators.

Bisperoxides obtained by the above processes can be purified by monoperoxides, present as by-products, according to the process of USP 3,584,059 which is entirely incorporated by reference in, the present description.

In the examples DUTRAL CO 054 ® means an E/P copolymer containing from 42 to 48% by weight of propylene; RIBLENE CF 2203 ® means a low-density polyethylene; ANOX HB ® means 2,2,4-trimethyl.1,2-dihydrochinoline polymer.

The following examples are given with illustrative but not limitative purpose of the present invention.

### EXAMPLE 1/a - Meta/para PEROXIMON 180 synthesis.

1 mole of benzol was alkylated with 1.5 moles of propylene, in the presence of AlCl₃ and according to usual reaction of Friedel-Kraft, so obtaining a mixture of benzene, cumene, diisopropylbenzene, tri-isopropylbenzene.
The mixture was rectified to obtain meta-para-diisopropylbenzene. This mixture was then oxidized by usual techniques with air so obtaining an oxidized mixture containing meta and para diisopropylbenzene, meta arid para monohydroperoxide of the diisopropylbenzene, meta and para dihydroperoxide of the diisopropylbenzene.
The meta and para dihydroperoxide were extracted from said oxidized mixture by an aqueous solution of soda.
The extracted product was reduced by sodic sulphydrate, according to usual techniques, by this way all hydroperoxidic groups (-O-O-H) were transformed into alcoholic groups, so obtaining an aqueous suspension of the dialcohol of formula (IV), constituted by 35% of the para isomer and by 65% of the meta isomer.
647 g of an heptan solution containing 1.84 moles of tert.amylhydroperoxide were introduced in a glass-flask, equipped with stirrer and thermometer, then 174 g of the dialcohol of formula (IV) containing 0.53 mole of the meta isomer, 0.28 mole of the para isomer and 0.97 mole of water were added.
331 g of an aqueous solution containing 70% by weight of sulphuric acid were gradually added during 60 minutes, at 5-8°C and under nitrogen stream; then the solution was kept at 5-8°C for two hours under stirring.
Then the aqueous phase was separated in a separatory funnel, and two washings were effected with 500 cm³ each time (at 60°C) with an aqueous solution of soda at 10% by weight; then two washings with 200 cm³ each time of deionized water.
After elimination of the heptane by distillation in steam current and after anhydrification under vacuum at 65°C, 193 g of a liquid peroxidic mixture were obtained having an active oxygen content equal to 6.97% by weight, a purity degree of 81% by weight (as total peroxidic content), a density of 0.933 g/cm³ and a viscosity (at 25°C) of 97 mPa.s.
The half-points of the mixture (by thermic decomposition) were 107°C (at 10 hours) and 178°C (at 1 minute); the gas-chromatographic analysis showed the following composition:
- meta PEROXIMON 180 45% by weight
- para PEROXIMON 180 36% by weight.
The rest were by-products.

### ESEMPIO 1/b - Meta PEROXIMON 180 synthesis.

644 g of an heptan solution containing 1.84 moles of tert.amylehydroperoxide were introduced in a glass-flask, equipped with stirrer and thermometer, then 174 g of dialcohol of formula (IV) were added, containing 0.807 moles of the pure meta isomer and 0.98 moles of water.
Then 331 g of an acqueous solution containing 70% by weight of sulphuric acid were gradually added, during 60 minutes, at 5-8°C and under nitrogen stream; the solution was kept at 5-8°C for two hours under stirring.
Then the aqueous phase was separated in a separating funnel and two washings were effected, with 500 cm³ for each time (at 60°C) of an aqueous solution of soda at 10% by weight, followed by other two washings with 200 cm³ for each time of deionized water.
After elimination of heptane under steam current, and after anhydrification under vacuum at 65°C, 178 g of peroxidic mixture were obtained having an active oxygen content of 7.01% by weight, a purity degree of 81% by weight (as total peroxidic content), a density of 0.931 g/cm³ and a viscosity (at 25°C) of 74.47 mPa.s.
The rest were by-products.

### EXAMPLE 1/c - Para PEROXIMON 180 synthesis.

644 g of an heptanic solution containing 1.84 mole of tert.amylehydroperoxide were introduced in a glass-flask, equipped with stirrer and thermometer, then 174 g of the dialcohol of formula (IV) were added, containing 0.807 moles of pure para isomer and 0.98 moles of water.
Then 331 g of an acqueous solution containing 70% by weight of sulphuric acid were gradually added, during 60 minutes, at 5-8°C under nitrogen stream; and the solution was maintained at 5-8°C for two hours under stirring.
Then the aquesous phase was separated in a separating funnel and two washings were effected, with 500 cm ³ for each time (at 60°C) of aqueous solution of soda at 10% by weight, followed by other two washings with 200 cm³ for each time of deionized water.
After heptane elimination by distillation in steam current, and after anhydrification under vacuum at 65°C, 202 g of peroxidic mixture were obtained with an active oxygen content equal to 7.02% by weight, a purity degree of 81% by weight (as total peroxidic content), a density of 0.935 g/cm³ and a viscosity (at 25°C) di 109.1 mPa.s.
The rest were by-products.

### EXAMPLE 1/d (comparison test)

### Applicative test of meta/para PEROXIMON 180.

100 parts by weight of an ethylene-propylene elastomeric copolymer, commercially known as DUTRAL CO 054^{R}, were mixed with 0.3 parts by weight of sulphur, 5 parts by weight of ZnO and 50 parts by weight of carbon black.
2.26 parts by weight of the peroxidic mixture of example 1/a were added and the resulting mixture was homogenized in a calender for a time of 5 minutes; the product from the calender showed the following properties:
a) ODR curve data at 170°C (oscillation arc = 3°; oscillation frequency 100 cycles/minute):

| | |
|---|---|
| MH | = 74.5 pounds * inches |
| ts10 | = 150 seconds |
| t90 | = 672 seconds |

The ODR curve (Oscillating Disc Rheometer) can be obtained by the aid of a rotating disc rheometer, under the ASTM-D-2084-71T rules.
The times are reported in abscissa and in ordinates the torque couple (pounds.inches) measured by dinamometer, opposed by the polymer to the disk rotation. It was found that the maximum reticulation density had a torque maximum value (EH = 74.5 pounds.inches) which no more changed with the time.
The expressions t90 and ts10 mean respectively the time occurring to reach a torque couple equal to 90% of the maximun torque couple and the time occurring to reach a level of 10 pounds.inches over the ODR curve minimum.
b) "Scorching" times at Mooney viscosimeter (135°C):

| | |
|---|---|
| t2 | = 588 seconds |
| t5 | = 930 seconds |

By "scorching" it is intended the undesired premature vulcanization occurring during the compound extrusion, before the exit from the extruder die; often said premature vulcanization often causes an interruption of the operations.
For scorching time t2 or t5 (at Mooney viscosimeter) it is intended the time occurring to reach an increase of the minimum viscosity value equal to 2 or to 5 Mooney units respectively.
The viscosity was determined by a Mooney viscosimeter at cutting disc (ASTM D 1646-81 rules).

### EXAMPLE 1/e (comparison test)

### Applicative test meta/para PEROXIMON 180.

100 parts by weight of polyethylene, commercially known as RIBLENE CF 2203® were mixed with 0.5 parts by weight of ANOX HB® and 0.5 parts by weight of TAC (triallyl cyanurate). 2.19 parts by weight of the peroxidic mixture of example 1/a were added and the resulting compound was homogenized in a calender, the product of the calender showed the following properties:
a) ODR curve data at 170°C (oscillation arc = 1°)

| | |
|---|---|
| MH | = 16.93 pounds * inches |
| ts10 | = 135 seconds |
| t90 | = 950 seconds |

b) "Scorching" times at Mooney viscosimeter (135°C):

| | |
|---|---|
| t5 | = 1034 seconds |
| t10 | = 1236 seconds. |

### EXAMPLE 2 - Binary mixtures PEROXIMON 180/PEROXIMON F

Meta/para PEROXIMON 180 of example 1/a was mixed with meta/para PEROXIMON F of usual industrial production, formed by 35% by weight of para isomer and of 65% by weight of meta isomer, in a weight ratio of 4:1, 3:2, 1:1, 2:3, 1:4.
Among these mixtures only the ones containing more than 65% by weight of meta/para PEROXIMON 180 remained liquid at room temperature (15°C).

| Mixture (a) | A | B | C | D | E |
|---|---|---|---|---|---|
| meta/para PEROXIMON 180 | 80% | 60% | 50% | 40% | 20% |
| meta/para PEROXIMON F | 20% | 40% | 50% | 60% | 80% |
| Physical status at 15°C | L | S | S | S | S |
| (L = liquid, S = solid, % is by weight). | | | | | |

In a similar way the meta PEROXIMON 180 of example 1/b was mixed with meta PEROXIMON F, of usual industrial production, in a weight ratio of 4:1, 3:2, 1:1, 2:3, 1:4.
Of these mixtures only those ones containing more than 65% by weight of meta PEROXIMON 180 remained liquid at room temperature (15°C).

| Mixture (b) | F | G | H | I | L |
|---|---|---|---|---|---|
| meta PEROXIMON 180 | 80% | 60% | 50% | 40% | 20% |
| meta PEROXIMON F | 20% | 40% | 50% | 60% | 80% |
| Physical status at 15°C | L | S | S | S | S |

The para PEROXIMON 180 of example 1/c was mixed with meta PEROXIMON F, of usual industrial production, in a weight ratio of 4:1, 3:2, 1:1, 2:3, 1:4.
Only the mixtures containing more than 65% by weight of meta PEROXIMON 180 remained liquid at room temperature (15°C).

| Mixture (c) | M | N | O | P | Q |
|---|---|---|---|---|---|
| para PEROXIMON 180 | 80% | 60% | 50% | 40% | 20% |
| meta PEROXIMON F | 20% | 40% | 50% | 60% | 80% |
| Physical status at 15°C | L | S | S | S | S |

Only the mixtures with 65% of PEROXIMON 180 resulted liquid at the temperature of 15°C.

### Applicative tests of the mixture of example 2:

### Example 2/a

100 parts by weight of an elastomeric ethylene-propylene copolymer, commercially known as DUTRAL CO 054^{R} were mixed with 0.3 parts by weight of sulphur, 5 parts by weight of ZnO and 50 parts by weight of carbon black.
2.20 parts by weight of the peroxidic composition of mixture 2/A were added to the mixture and the resulting mixture was homogenized in, a calender; the product of the calender showed the following properties:
a) ODR curve data at 170°C (oscillation arc = 3°; oscillation frequency 100 cycles/minute).

| | |
|---|---|
| MH | = 78.1 pounds * inches |
| ts10 | = 140 seconds |
| t90 | = 699 seconds. |

b) "Scorching" times at Mooney viscosimeter (135°C):

| | |
|---|---|
| t2 | = 590 seconds |
| t5 | = 890 seconds. |

### Example 2/b

100 parts by weight of polyethylene, commercially known as RIBLENE CF 2203® were mixed with 0.5 parts by weight of ANOX HB® and 0.5 parts by weight of TAC (triallyl cyanurate). 2.16 parts by weight of the peroxidic mixture of mixture 2/A were added to the composition and the resulting compound was homogenized in a calender; the product of the calender showed the following properties:
a) ODR curve data at 170°C (oscillation arc = 1°)

| | |
|---|---|
| MH | = 16,99 pounds * inches |
| ts10 | = 136 seconds |
| t90 | = 952 seconds. |

b) "Scorching" times at Mooney viscosimeter (135°C):

| | |
|---|---|
| t5 | = 1038 seconds |
| t10 | = 1240 seconds. |

### Example 2/c

100 parts by weight of an elastomeric ethylene-propylene copolymer, commercially known as DUTRAL CO 054® were mixed with 0.3 parts by weight od sulphur, 5 parts by weight of ZnO and 50 part by weight of carbon black.
2.10 parts by weight of the peroxidic mixture of mixture 2/M were added and the resulting compound was homogenized in a calender; the product of the calender showed the following properties:
a) ODR curve data at 170°C (oscillation arc = 3°; oscillation frequency 100 cycles/minute).

| | |
|---|---|
| MH | = 78 pounds * inches |
| ts10 | = 138 seconds |
| t90 | = 695 seconds. |

b) "Scorching" times at Mooney viscosimeter (135°C):

| | |
|---|---|
| t2 | = 588 seconds |
| t5 | = 890 seconds. |

## Claims

1. Liquid peroxidic compositions containing essentially the following components per 100 parts by weight of composition:
- from 65 to 95 parts by weight of bis(alpha-t-amylperoxyisopropyl)-benzene (I), 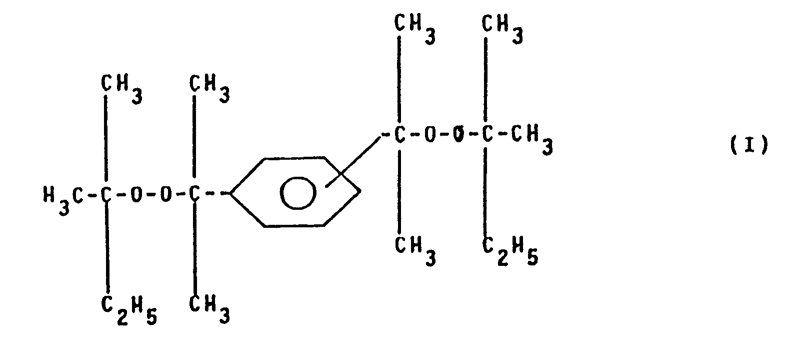 the two substituents being in meta and/or para position with respect to the benzen ring, the meta/para ratio being preferably comprised between 1 and 2.0; and
- from 5 to 35 parts by weight of bis-(alpha-t-butyl-peroxy-isopropyl)-benzene (II), 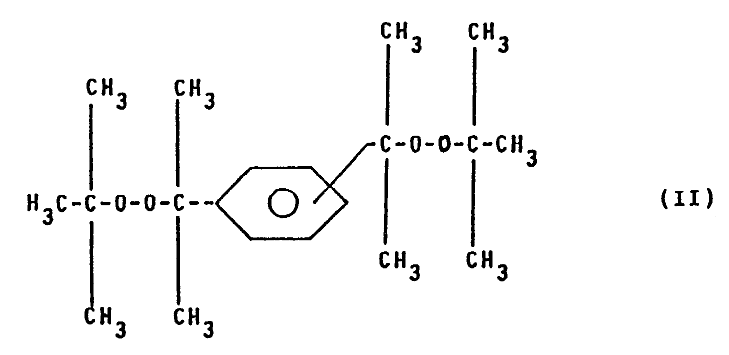 the two substituents being in meta and/or para position with respect to the benzene ring, the meta/para ratio preferably ranging between 1.5 and 2;
the para(I)/para(II) and meta(I)/para(II) compositions being excluded.

2. Liquid peroxidic compositions according to claim 1, wherein in (I) the meta/para ratio is comprised between 1 and 2.0 and in (II) the meta/para ratio ranges between 1.5 and 2.

3. Liquid peroxidic compositions according to claims 1, 2, wherein the (I) and (II) components are in the form of meta/para mixtures.

4. Liquid peroxidic compositions according to claim 1, wherein the component (I) is in the para form and (II) in the meta form.

5. Use of the liquid peroxidic compositions according to claims 1-4, in the cross-linking of polymers.

6. Use of the liquid peroxidic compositions according to claim 5, wherein the polymer is selected from polyethylene, ethylene-propylene elastomers optionally containing a dienic monomer.

## Patentansprüche

1. Flüssige peroxidische Zusammensetzungen, welche im wesentlichen die folgenden Komponenten je 100 Gewichtsteile der Zusammensetzung enthalten:
- 65 bis 95 Gewichtsteile Bis(alpha-t-amylperoxy-isopropyl)-benzol (I), 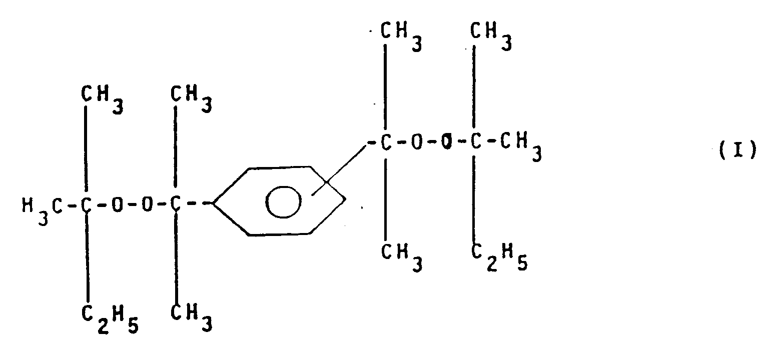 wobei die beiden Substituenten in der meta- und/oder para-Position des Benzolringes sind, das meta/para-Verhältnis vorzugsweise zwischen 1 und 2,0 umfaßt; und
- 5 bis 35 Gewichtsteile Bis(alpha-t-butyl-peroxy-isopropyl)-benzol (II), 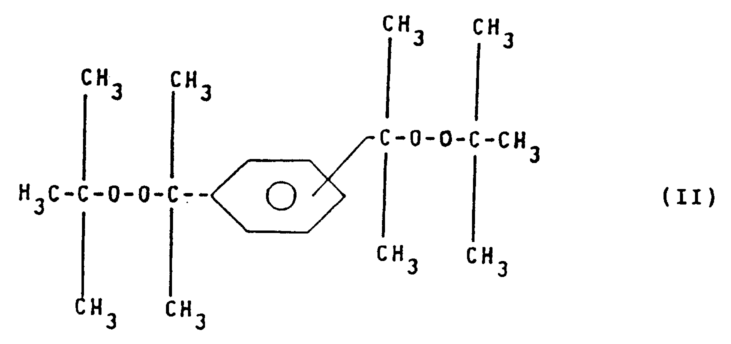 wobei die beiden Substituenten in der meta- und/oder para-Position des Benzolringes sind, das meta/para-Verhältnis vorzugsweise zwischen 1,5 und 2,0 liegt;
ausgenommen die Zusammensetzungen para (I)/para (II) und meta (I)/para (II).

2. Flüssige peroxidische Zusammensetzungen nach Anspruch 1, wobei in (I) das meta/para-Verhältnis zwischen 1 und 2,0 umfaßt und in (II) das meta/para-Verhältnis zwischen 1,5 und 2 liegt.

3. Flüssige peroxidische Zusammensetzungen nach Anspruch 1, 2, wobei die Komponenten (I) und (II) die Form eines meta/para-Gemisches aufweisen.

4. Flüssige peroxidische Zusammensetzungen nach Anspruch 1, wobei die Komponente (I) in der para-Form und die Komponente (II) in der meta-Form vorliegt.

5. Verwendung der flüssigen peroxidischen Zusammensetzungen nach den Ansprüchen 1-4 zum Vernetzen von Polymeren.

6. Verwendung der flüssigen peroxidischen Zusammensetzungen nach dem Anspruch 5, wobei das Polymer ausgewählt wird aus Polyethylen, Ethylen-Propylen-Elastomeren, die gegebenenfalls ein Dien-Monomer enthalten.

## Revendications

1. Compositions peroxydiques liquides, contenant essentiellement les composants suivants, pour 100 parties en poids de composition :
- de 65 à 95 parties en poids de bis(alpha-t-amylperoxy-isopropyl)-benzène (I) : 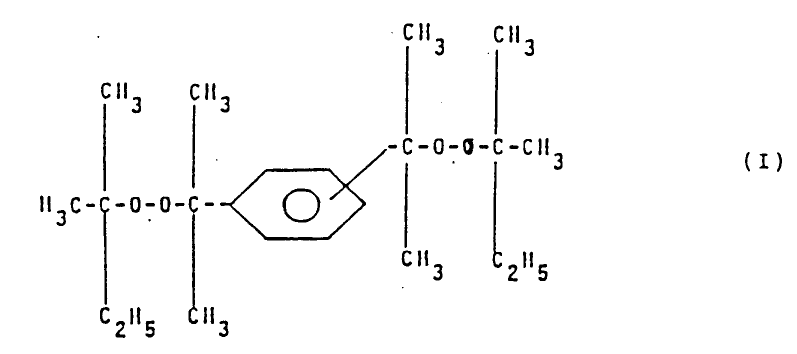 les deux substituants étant en position méta et/ou para par rapport au noyau benzène, le rapport méta/para étant, de préférence, compris entre 1 et 2,0 ; et
- de 5 à 35 parties en poids de bis-(alpha-t-butyl-peroxy-isopropyl)-benzène (II) : 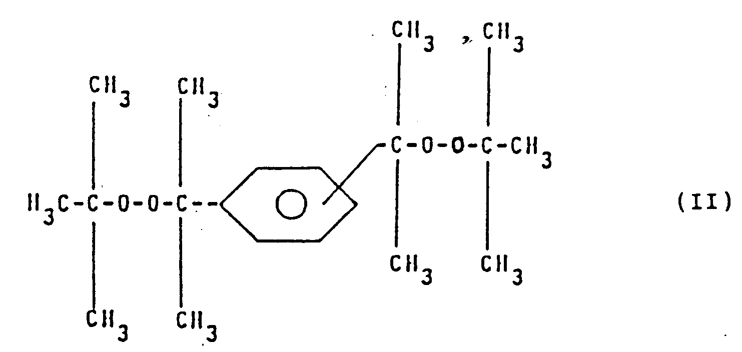 les deux substituants étant en position méta et/ou para par rapport au noyau benzène, le rapport méta/para étant, de préférence, compris entre 1,5 et 2 ;
les compositions para (I)/para (II) et méta (I)/para (II) étant exclues.

2. Compositions peroxydiques liquides selon la revendication 1, dans lesquelles, dans (I), le rapport méta/para est compris entre 1 et 2,0, et, dans (II), le rapport méta/para se situe entre 1,5 et 2.

3. Compositions peroxydiques liquides selon l'une des revendications 1 et 2, dans lesquelles les composants (I) et (II) se présentent sous la forme de mélanges méta/para.

4. Compositions peroxydiques liquides selon la revendication 1, dans lesquelles le composant (I) se présente sous la forme para, et (II), sous la forme méta.

5. Utilisation des compositions peroxydiques liquides telles que définies à l'une des revendications 1 à 4, dans la réticulation de polymères.

6. Utilisation des compositions peroxydiques liquides selon la revendication 5, dans laquelle le polymère est choisi parmi le polyéthylène, les élastomères éthylène-propylène contenant facultativement un monomère diénique.
